# EUROPEAN PATENT APPLICATION

(11) **EP 2 746 261 A1**
(43) Date of publication of application: **25.06.2014**
(21) Application number: 14160850.5
(22) Date of filing: 02.09.2009
(51) Int. Cl.: C07D 233/58, C07D 235/08

(54) **Process for synthesis of imidazolium and benzimidazolium surfactants and their use in clays and nanocomposites**

(30) Priority: 03.09.2008 US 203786
(62) Divisional of application: 09792164.7
(71) Applicant: SABIC Innovative Plastics IP B.V., 4612 PX Bergen op Zoom (NL)
(72) Inventor: Karanam, Sreepadaraj, NL-4611 JX Bergen op Zoom (NL); Berti, Corrado, I-48022 Lugo (IT); Binassi, Enrico, I-40134 Bologna (IT); Brunelle, Daniel, Joseph, Burnt Hills, NY New York 12027 (US); Colonna, Martino, I-40122 Bologna (IT); Fiorini, Maurizio, I-40011 Anzola Emilia (IT)
(74) Representative: Carpintero Lopez, Francisco

(57) **Abstract**

Compositions of a semicrystalline thermoplastic and a dispersed phase of an organically modified clay are disclosed, as well as a process for making the organically modified clay, which comprises contacting the clay with at least one imidazolium salt having two long alkyl chains, or a benzimidazolium salt having two long alkyl chains in the presence of a solvent, and recovering the organically modified clay having a d-spacing of clay intercalates of at least 28Å and thermal stability at 300°C.

## Description

### BACKGROUND OF THE INVENTION

The present invention relates to an improved process for the synthesis of dialkyl chain imidazolium and benzimidazolium organic modifiers with high thermal stability, high organic character and corresponding modified nanoclays with high d-spacing to prepare nanocomposites of various polymer composites, especially semicrystalline thermoplastics, such as poly(butylene terephthalate) (PBT).

Nanocomposites are a new class of composites that are particle-filled polymers for which at least one of the dimensions of the dispersed phase is in the nanometer range, typically 1-20 nm. Polymer layered nanocomposites often have superior physical and mechanical properties over their microcomposites counterparts, including improved modulus, reduced gas permeability, flame retardancy and improved scratch resistance. Moreover, the nanoscale dispersion of the filler does not give rise to the brittleness and opacity typical of composites.

Polymeric, intercalation-type nanocomposites have been the subject of extensive research over the past decade. Much of the work in this area has been focused on polymeric nanocomposites derived from layered silicates such as montmorillonite clay.

Polymer nanocomposites comprising a semicrystalline polymer matrix are particularly attractive due to the dramatic improvement in heat distortion temperature and modulus provided by the nanoparticles reinforcement and the high flow character inherent to most commodity semicrystalline thermoplastics such as nylon-6, nylon-6,6, poly(butylene terephthalate), poly(ethylene terephthalate), polypropylene, polyethylene, etc. Because of these desirable characteristics, semicrystalline polymer nanocomposites have been shown to be well-suited for application as injection moldable thermoplastics.

Since melt intercalation processes require high processing temperature, it is of fundamental importance that the organically modified clay does not degrade during the nanocomposite preparation. Quaternary alkyl ammonium salts are the most commonly used to modify the clays. However, those salts start degrading below 240°C that is the temperature used during poly(butylene terephthalate) (hereinafter, "PBT") nanocomposite preparation in twin screw extruders by the melt compounding method. For this reason, the development of new clays with more stable surfactants and/or organic modifiers would be highly desirable for use in the preparation of nanocomposites with thermoplastic polyesters and polycarbonates that present extrusion temperature above 240°C.

Homogeneous nanoscale dispersion of clays is needed to obtain nanocomposites with improvement in mechanical, thermal, and flame properties. It is necessary to match the polarity difference between the polar clay and non-polar polymer matrix to obtain nanocomposites with well-dispersed clays. (*See* J. Polym. Sci.: part B: Polym. Phys., 44, 1040-1049). In light of these needs, it would be highly desirable to develop organic modifiers with higher thermal stability and high organic character to obtain corresponding modified clays with high d-spacing.

Attempts have been made to use imidazolium salts to improve the thermal stability of functionalized clay. (*See* H, LeCompte K, Hargens L, McEwen BA. Thermochim Acta 2000; 357; 97-105; Begg G, Grimmett RM, Wethey DP, Aust. J. Chem. 1977; 30; 2005-15). Information relevant to attempts to assess the potential of using the imidazolium molten salts as a replacement for the alkyl ammonium surfactant on the formation of nanocomposites can be found in:
(1) Davis RD, Sheilds J, Harris Jr RH., Davis C, et al., Chem Mater 2002; 14:3776-3785;
(2) Davis CH, Mathias LJ, Gilman JW, Schiraldi JR, Trulove P, Sutto TE, ct al., J. Polym. Sci. Polym. Phys. 2002; 40(23):2661-2666;
(3) Maupin PII, Gilman JW, bourbigot S, Harris JR RH, Bellayer S, Bur AJ et al. Macromol. Rapid Commun. 2004; 25(7):788-792;
(4) Wang ZM, Chung TC, Gilman JW, Manias E. J. Polym. Sci. part B 2003; 41:3173-3187;
(5) Bottino FA, Fabbri E, Fragala IL, Malandrino G, Orestano A, Pilati F, et al. Macromol. Rapid Commun. 2003; 24: 1079-1084; and
(6) Kommann X, Thomann R, Finter J, Berglund LA. Polym. Eng. Sci 2002; 42:1815-1822.

Attempts have been made to use onium salts as clay modifiers in PBT and poly(phenylene ether) nanocomposites. Information regarding such attempts can be found in US Patent 5,707,439 to Takekoshi et al. (hereinafter, "the '439 patent"). The '439 patent is unconcerned with molecular weight retention of corresponding polyester nanocomposites, and only nanocomposites having a low molecular weight retention are disclosed.

Attempts have been made to use other compalibilizers in the preparation of polymer/layered silicate nanocomposites. Information relevant to attempts to use imidazolium salts as clay modifiers can be found in:
(1) Gilman J.W., Awad W.H., Davis R.D., Delong H.C. Chem Mater. 2002, 14, 3776; and
(2) Awad W.H., Gilman J.W., Nyden M., Harris R.H., Sutto T.E., Callahan J., Trulove P.C., DeLong H.C., Fox D.M. Thermochimica Acta, 409(1), 3-11 (2004).
   However, these references suffer from the following disadvantage: an onset decomposition temperature of only 320-340°C in thermal, gravimetric analysis (TGA) was observed for imidazolium modified montmorillonite. In other words, the imidazolium modified montmorillonite lacked thermal stability above 320-340°C. In comparison, onset decomposition temperature for hydrogenated tallow ammonium modified montmorillonite is 220-250°C.
Information relevant to attempts to utilize imidazolium surfactants bearing one C₁₆ alkyl chain for the preparation of poly(ethylene terephthalate) nanocomposites, as well as, poly(ethylene naphthalate) and other polymer matrixes can be found in:
   (1) Davis C. H., Mathias L.J., Gilman J.W., Schiraldi D.A., Shields, J.R., Trulove, P., Sutto T.E., Delong H.C. J. Polym. Sci., Part B: Polym. Phys. (2002), 40(23), 2661-2666;
   (2) Malhotra S.V., Kim N.H., Xanthos M. Abstracts of Papers, 231st ACS National Meeting, Atlanta, March 26-30, 2006;
   (3) Zhao J., Morgan A.B., Harris J.D. Polymer (2005), 46(20), 8641-8660;
   (4) Morgan A.B., Harris J.D., PMSE Preprints (2005), 92 412-413;
   (5) He A., hu H., Huang Y., Dong J., Han C.C. Macromol. Rapid Comm. (2004), 25(24), 2008-2013;
   (6) Bottino F.A., Fabbri, e., Fragala, I.L., Malandrino G., Orestano A., Pilati F., Pollicino A. Macromol. Rapid Comm. (2003), 24(18), 1079-1084; and
   (7) Kono K., Kido N., Nishio R. (Teijin Ltd., Japan). Jpn. Kokai Tokkyo Koho (2005) JP 2005298751 A2 20051027.
   However, the clays produced according to the references suffer from the disadvantage of a d-spacing below 25-26 Angstroms (Ǻ). In other words, the clays suffer from the disadvantage of a d-spacing below that of the widely used hydrogenated tallow (HT)-ammonium modified clays.

Information relevant to attempts to utilize imidazolium salts bearing two C₁₀ alkyl chains can be found in:
(1) Chua, Yang Choo; Wu, Shucheng, Lu, Xuehong. Journal of Nanoscience and Nanotechnology (2006), 6(12), 3985-2988; and
(2) Chua, Yang Choo; Lu, Xuehong; Wan, Tong. Journal of Polymer Science, Part B: Polymer Physics (2006), 44(7), 1040-1049.
However, both references suffer from the disadvantage that the d-spacing of the high organic character clays was 25 Ǻ. This d-spacing is below that of HT-ammonium modified clays. Both references also suffer from the disadvantage that the thermal stability of the clay obtained was lower than that reported for monoalkyl imidazolium clays.

Information relevant to attempts to use imidazolium bearing two C₁₆ alkyl chains can be found in: Wang, Z.M.; Chung, T.C.; Gilman, J.W., Manias, E.J. of Polym. Sci. e, Part B: Polym. Phys. (2003), 41(24), 3173-3187. However, this reference suffers from the disadvantage that the d-spacing of the clay was 24Ǻ, which is below that of HT-ammonium modified clays. Since the d-spacing of this clay is the same as imidazolium with just one alkyl chain, this result is not in agreement with a reference mentioned above, i.e., Chua, Yang Choo; Wu, Shucheng; Lu, Xuehong. Journal of Nanoscience and Nanotechnology (2006), 6(12), 3985-3988. According to that reference, dialkyl imidazolium organic modifiers have higher d-spacing compared to corresponding monoalkyl modifiers. Thus, the art is in a state of confusion and disarray with regard to the effects of dialkyl imidazolium organic modifiers compared with monoalkyl imidazolium organic modifiers.

Information relevant to attempts to provide a d-spacing larger than 30Ǻ can be found in Fox, Douglas M.; Maupin, Paul H.; Harris, Richard H., Jr.; Gilman, Jeffrey W.; Eldred, Donald V.; Katsoulis, Dimi; Trulove, Paul C.; DeLong, Hugh C. Langmuir (2007), 23(14), 7707-7714. This reference suffers from the disadvantage that the exchange efficiency of ion-exchange reaction with clay was limited and dependent on the solvent used in the reaction. Furthermore, actual industrial use of the POSS-based organic modifier is cost prohibitive.

For the foregoing reasons, there is an unmet need to develop improved methods for making organically modified clays having relatively higher d-spacing.

For the foregoing reasons, there is an unmet need to develop organically modified clays having thermal stability at relatively higher temperatures, e.g., 300°C or higher.

For the foregoing reasons, there is an unmet need to develop poly(butylene terephthalate) compositions containing nanometer-sized, organically modified clays having relatively higher d-spacing of clay intercalates and thermal stability at high temperatures.

### BRIEF SUMMARY

In a first embodiment, the invention provides a process for making an organically modified clay. The process comprises contacting the clay with at least one imidazolium salt according to formula I, or a benzimidazolium salt according to formula II. 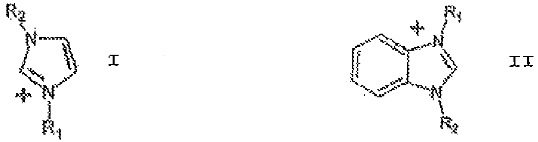 In this embodiment of the present invention, R₁ and R₂, which may be the same or different, are each a C₁₂-C₂₅ alkyl group in the presence of a solvent. The process further comprises recovering the organically modified clay having a d-spacing of clay intercalates of at least 28Å and thennal stability at 300°C or above.

In a second embodiment, the invention provides a product produced by a process for making an organically modified clay. The process comprises contacting the clay with at least one imidazolium salt, according to formula I, or a benzimidazolium salt, according to formula II; and recovering the organically modified clay having a d-spacing of clay intercalates of at least 28Å and thermal stability at 300°C. In this embodiment, R₁ and R₂, which may be the same or different, are each a C₁₂-C₂₅ alkyl group in the presence of a solvent.

In a third embodiment, the invention provides a composition comprising a semicrystalline thermoplastic and a dispersed phase of an organically modified clay. In this embodiment, the clay is organically modified with an imidazolium or benzimidazolium salt such that the modified clay has a d-spacing of clay intercalates of at least 28Å and thermal stability at 300°C.

In a fourth embodiment, the invention provides a composition comprising a poly(butylene terephthalate) having a dispersed phase of a nanometer sized, organically modified clay, wherein the clay has a d-spacing of clay intercalates of at least 30Å and a thermal stability of at least 350°C.

### BRIEF DESCRIPTION OF THE FIGURES

Figure 1 shows the thermal stability of clays modified with ammonium and imidazolium surfactants in N₂ at 10°C/min.
Figure 2 shows an XRD of clays modified with imidazolium and benzimidazolium surfactants.
Figure 3 shows a comparison of an XRD of D-SAB with two C16 and with two C18 chains.
Figure 4 shoes a comparison of storage moduli of nanocomposites obtained using clays modified with ammonium and imidazolium clays.

### DETAILED DESCRIPTION

The invention is based on the discovery that it is now possible to make clays having relatively higher d-spacing with certain organic modifiers under certain conditions. It is advantageous to obtain such a relatively higher d-spacing in clays, because the relatively higher d-spaying helps to obtain well-dispersed clays in nanocomposites, which in turn, helps obtain nanocomposites with better physical properties. The clays have excellent thermal stability and are particularly suitable for making polyester-based nanocomposites, among other nanocomposites. Compositions containing such clays exhibit homogeneous nanoscale dispersion of clays, which is very important to obtain nanocomposites with excellent improvement in mechanical, thermal, and flame properties.

The present invention may be understood more readily by reference to the following detailed description of preferred embodiments of the invention and the examples included therein. In the following specification and the claims that follow, reference will be made to a number of terms that shall be defined to have the following meanings.

The singular forms "a," "an," and "the" include plural referents unless the context clearly dictates otherwise.

"Optional" or "optionally" means that the subsequently described event or circumstance may or may not occur, and that the description includes instances where the event occurs and instances where it does not.

"Dispersion" or "dispersed" refers to the distribution of the organoclay particles in the polymer matrix.

"Intercalated" or "intercalate" refers to a higher degree of interaction between the polymer matrix and the organoclay as compared to mere dispersion of the organoclay in the polymer matrix. When the polymer matrix is said to intercalate the organoclay, the organoclay exhibits an increase in the interlayer spacing between adjacent platelet surfaces as compared to the starting organoclay.

"Delamination" refers to the process of separation of ordered layers of clay platelets through the interaction of the organoclay with the polymer matrix.

"Exfoliate" or "exfoliated' shall mean platelets dispersed mostly in an individual state throughout a polymer matrix material. Herein, "exfoliated" is used to denote the highest degree of separation of platelet particles. "Exfoliation" refers to the process by which an exfoliate is formed from an intercalated or otherwise dispersed organoclay within a polymer matrix.

"Nanocomposite(s)" and "nanocomposite composition(s)" refer to a polymer or copolymer having dispersed therein a plurality of individual clay platelets obtained from a layered clay material, wherein the individual particle sizes are less than about 100 nm.

"Matrix polymer," "bulk polymer," or "bulk matrix polymer" refers to the continuous phase of a nanocomposite.

"Telechelic polymer" refers to a linear polymer whose end groups are functionalized with a suitable organic functional group such as carboxylates, sulfonates and the like.

The terms "BRABENDER^{®}" or "BRABENDER^{®} mixer" refer to physical blenders, mixers, or extruders available from BRABENDER^{®} GmbH and Co. KG.

Other than in the operating examples or where otherwise indicated, all numbers or expressions referring to quantities of ingredients, reaction conditions, and the like, used in the specification and claims are to be understood as modified in all instances by the term "about." Various numerical ranges are disclosed in this patent application. Because these ranges are continuous, they include every value between the minimum and maximum values. Unless expressly indicated otherwise, the various numerical ranges specified in this application are approximations. The endpoints of all ranges directed to the same component or property are inclusive of the endpoint and independently combinable.

As used in the specification and claims, and unless otherwise specifically noted, all references to molecular weight (Mw) are to weight average molecular weight.

In a first embodiment, the invention provides a process for making an organically modified clay. The process comprises contacting the clay with at least one imidazolium salt according to formula I, or a benzimidazolium salt according to formula II in the presence of a solvent. 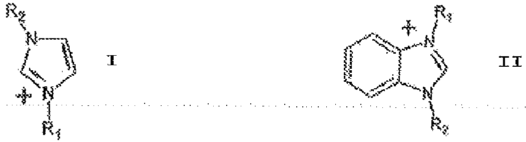 R₁ and R₂, which may be the same or different, are each a C₁₂-C₂₅ alkyl group. In a preferred embodiment of the process, R₁ and R₂, which may be the same or different, are each a C₁₄-C₂₀ alkyl group. In a particularly preferred embodiment of the process, R₁ and R₂, which may be the same or different, are a C₁₆-C₁₈ alkyl group. It is often preferable that R₁ and R₂ are the same. In a particularly preferred embodiment of the process, the clay is contacted with the imidazolium salt and R₁ and R₂ arc the same.

According to the process of the present invention the clay is contacted with the at least one imidazolium salt or benzimidazolium salt in the presence of a solvent. In a preferred embodiment of the process, the solvent is methanol.

The process further comprises recovering the organically modified clay having thermal stability at 300°C. In a preferred embodiment of the process, the organically modified clay has a thermal stability at a temperature from 300 to 350°C. More preferably the organically modified clay has a thermal stability at a temperature from 310 to 350°C. More preferably the organically modified clay has a thermal stability at a temperature from 320 to 350°C. More preferably the organically modified clay has a thermal stability at a temperature from 330 to 350°C. More preferably the organically modified clay has a thermal stability at a temperature from 340 to 350°C. In a particularly preferred embodiment of the process, the organically modified clay has thermal stability at a temperature that is greater than or equal to 350°C, more preferably at greater than or equal to 360°C, and most preferably at greater than or equal to 365°C.

The process further comprises recovering the organically modified clay having a d-spacing of clay intercalates of from of at least 28Å. In a preferred embodiment of the process, the d-spacing of the organically modified clay is greater than or equal to 30Å. More preferably the d-spacing of the organically modified clay is from 29-36Å. More preferably the d-spacing of the organically modified clay is from 30-36Å. More preferably the d-spacing of the organically modified clay is from 31-36Å. More preferably the d-spacing of the organically modified clay is from 32-36Å. More preferably the d-spacing of the organically modified clay is from 33-36Å. More preferably the d-spacing of the organically modified clay is from 34-36Å. More preferably the d-spacing of the organically modified clay is from 35-36Å.

In a particularly preferred embodiment of the process, R₁ and R₂ are each C₁₈ alkyl groups and the organically modified clay has a d-spacing of at least 36Å.

In a second embodiment, the invention provides a product produced by a process for making an organically modified clay. The process comprises contacting the clay with at least one imidazolium salt, according to formula I, or a benzimidazolium salt, according to formula II, in the presence of a solvent; and recovering the organically modified clay. R₁ and R₂, according to formula I and/or II, may be the same or different, and are each a C₁₂-C₂₅ alkyl group, more preferably a C₁₄-C₂₀ alkyl group, and even more preferably a C₁₆-C₁₈ alkyl group. The recovered organically modified clay has a d-spacing of clay intercalates of from 21-36 Å, preferably of at least 28Å. The recovered organically modified clay has a thermal stability at 300°C, preferably at greater than or equal to 300°C, more preferably at greater than or equal to 310°C, more preferably at greater than or equal to 320°C, more preferably at greater than or equal to 330°C, more preferably at greater than or equal to 340°C, more preferably at greater than or equal to 350°C, more preferably at greater than or equal to 360°C, and most preferably at greater than or equal to 365°C.

In a third embodiment, the invention provides a composition comprising a semicrystalline thermoplastic and a dispersed phase of an organically modified clay.

It is preferred that the semicrystalline thermoplastic is at least one selected from the group consisting of nylon-6, nylon-6,6, poly(butylene terephthalate), poly(ethylene terephthalate), polypropylene and polyethylene. In a further preferred embodiment of the composition the semicrystalline thermoplastic is one selected from the group consisting of polyesters and polyester ionomers. It is particularly preferred that the semicrystalline thermoplastic is one selected from the group consisting of polyester ionomers, and the polyester ionomers are random ionomers. It is also particularly preferable for the polyester ionomers to be telechelic.

In the third embodiment of the invention, which provides a composition comprising a semicrystalline thermoplastic and a dispersed phase of an organically modified clay, the clay is preferably organically modified with an imidazolium or benzimidazolium salt such that the modified clay has a d-spacing of clay intercalates of at least 28Å and thermal stability at 300°C or higher.

In a particularly preferred embodiment of the composition comprising a semicrystalline thermoplastic and a dispersed phase of an organically modified clay, as described above, the clay is one selected from the group consisting of montmorillonite, kaolin, illite and combinations thereof. It is preferred that the modified clay has a particle size in the range of from 1-50 nm, preferably from 1-20 nm. The modified clay is present in an amount of from 0.1 to 30% by weight, preferably present in an amount of from about 0.5 to about 15% by weight. The thermoplastic is present in an amount of from 75 to 99.9% by weight, preferably from about 85 to about 99.5% by weight.

In a preferred embodiment of the composition comprising a high temperature processable (melt temperature greater than 230°C) semicrystalline thermoplastic and a dispersed phase of an organically modified clay, as described above, the composition retains from 80-100% of its molecular weight, preferably from 90-100% of its molecular weight (Mw) measured via GPC when blended in a BRABENDER^{®} mixer for 10 minutes at 60 rpm. Most preferably the composition retains at least 92% of its molecular weight measured via GPC when blended in a BRABENDER^{®} mixer for 10 minutes at 60 rpm. It is also preferable that the composition has thermal stability at 300°C, preferably at greater than or equal to 300°C, more preferably at greater than or equal to 310°C, more preferably at greater than or equal to 320°C, more preferably at greater than or equal to 330°C, more preferably at greater than or equal to 340°C, more preferably at greater than or equal to 350°C, more preferably at greater than or equal to 360°C, and most preferably at greater than or equal to 365°C. Typically, the composition has thermal stability at a temperature ranging from 300 to 350°C. In a particularly preferred embodiment of the composition comprising a semicrystalline thermoplastic and a dispersed phase of an organically modified clay, as described above, the modified clay is present in an amount of from about 0.5 to about 25% by weight, preferably in an amount of from about 2 to about 10% by weight and the thermoplastic is present in an amount of from about 75 to about 100% by weight, preferably from about 90 to about 98% by weight.

In a fourth embodiment, the invention provides a composition comprising a poly(butyl terephthalate) having a dispersed phase of a nanometer sized, organically modified clay, wherein the clay has a d-spacing of clay intercalates is from 29-36Å. More preferably the d-spacing of clay intercalates is from 30-36Å. More preferably the d-spacing of clay intercalates is from 31-36Å. More preferably the d-spacing of clay intercalates is from 32-36Å. More preferably the d-spacing of clay intercalates is from 33-36Å. More preferably the d-spacing of clay intercalates is from 34-36Å. More preferably the d-spacing of clay intercalates is from 35-36Å. It is particularly preferred that the d-spacing of clay intercalates is at least 30Å.

The clay has a thermal stability thermal stability at 300°C, preferably at greater than or equal to 300°C, more preferably at greater than or equal to 310°C, more preferably at greater than or equal to 320°C, more preferably at greater than or equal to 330°C, more preferably at greater than or equal to 340°C, more preferably at greater than or equal to 350°C, more preferably at greater than or equal to 360°C, and most preferably at greater than or equal to 365°C.

In a preferred embodiment of the composition comprising a poly(butylene terephthalate) having a dispersed phase of a nanometer sized, organically modified clay, the dispersed phase of organically modified clay is in the range of from 1-50 nm, preferably from 1-20 nm.

The N,N-dialkyl imidazolium chloride salts utilized in the present invention can be synthesized by procedure illustrated below:

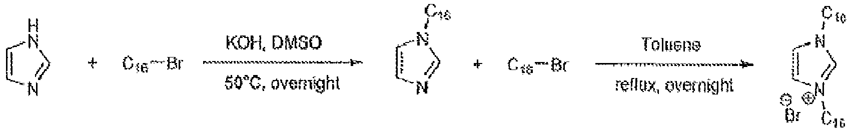

This synthesis procedure is described in more detail in Starikova, O.V.; Dolgushin, G.V.; Larina, L.I.; Komarova, T.N.; Lopyrev, V.A. ARKIVOC (Gainesville, FL, United States) (2003), (13), 119-124, which is hereby incorporated by reference.

For purposes of the present invention, the sodium montmorillonite (MMT) can be modified by ion exchange with a cation containing organic modifier with any suitable method. In one embodiment, the MMT can be modified by ion exchange with a modification of the procedure described in Awad W.H., Gilman J.W., Nyden M., Harris R.H., Sutton T.E., Callahan J., Trulove P.C., DeLong H.C., Fox D.M. Thermochimica Acta (2004), 409(1), 3-11, which is hereby incorporated by reference. In one embodiment, methanol can be used as a solvent for the dissolution of the imidazolium salt and a purification procedure in dichloromethane instead of ethanol can be performed. Clays can be prepared with imidazolium and benzimidazolium salts with one or two alkyl chains, for example:

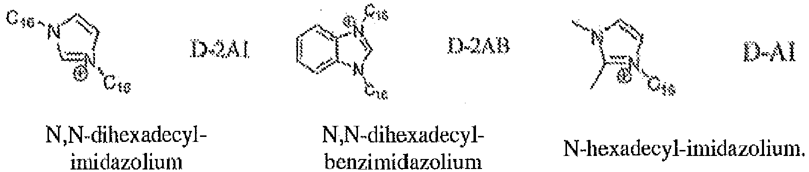

Clays modified with the various organic modifiers, can be characterized by X-ray diffraction (XRD) and thermal gravimetric analysis (TGA). For purposes of discussion, m the paragraph above, clays prepared with N,N-dihexadecyl-Imidazolium have been labeled "D-2AI," clays prepared with N,N-dihexadecyl-Benzinidazolium have been labeled "D-2AB," and clays prepared with N-hexadecyl-Iimdazolium have been labeled "D-AI." Note that D-AI clays are used for comparative purposes.

Table 1 shows the d-spacing and the thermal stability of imidazolium and benzimidazolium clays, which can be prepared according to the present invention compared with commercial HT montmorillonite, such as Dellite^{®} 72T.

| **Table 1** | | | |
|---|---|---|---|
| Clay | XRD analysis d-spacing (Ǻ) | TGA analysis Tₒₙₛₑₜ (°C) | TGA analysis Tₚₑₐₖ (°C) |
| D-2AI Clay (Imidazolium 2C16) | 31 or higher | 353 or higher | 428 or higher |
| D-2AB (Benzimidazolium 2C16) | 32 or higher | 367 or higher | 445 or higher |
| D-2AB (Benzimidazolium 2C18) | 36 or higher | 350 or higher | 410 or higher |
| Dellite^{®} 72T (HT ammonium) | 26 or higher | 258 or higher | 303 or higher |
| D-AI (imidazolium C16) | 21 or higher | 350 or higher | 420 or higher |

As reported in Table 1, the imidazolium clays can show at least a 100°C increase in thermal stability compared to standard ammonium clays, such as Dellite^{®} 72T. The benzimidazolium is slightly more stable compared to the imidazolium. Almost no difference between one and two alkyl chains is observed.

The nanocomposites can be prepared in a BRABENDER^{®} mixer using 3% sulfonated telechelic polybutylene terephthalate (PBT) and standard PBT as polymer matrix using 5% by weight of the clays. The blending temperature can be any suitable temperature, generally temperature is generally between 230 to 280°C. The residence time in the mixer can range from 5 to 15 minutes. In one embodiment, the residence time can be 10 minutes.

Nanocomposites containing PBT and clays made in accordance to the present invention exhibit almost no drop in molecular weight (Mw) of PBT after blending under various conditions, for example, blending for 10 minutes at 60 rpm in a BRABENDER^{®} mixer with 5% by weight of imidazolium and benzimidazolium clays. Using 10% of clay can give rise to a slight Mw drop while a more consistent drop can be observed using 5% w/w of HT ammonium (Dellite 72T) clay.

The final composites obtained according to the present invention can demonstrate thermal stability of 7-15°C higher than the starting polymers. The mechanical properties determined by Dynamic Mechanical Thermal Analysis (DMTA) and the morphology determined by Transmission Electron Microscopy (TEM) of the nanocomposites prepared using imidazolium modified clays do not differ from those of the nanocomposite obtained with ammonium modified clays, since also using the imidazolium and benzimidazolium surfactants a mainly exfoliated morphology and a consistent increase in heat distortion temperature have been obtained.

Advantageously, our invention now provides a new method for making organically modified clays having highly useful d-spacing characteristics and thermal stability at high temperatures. Our invention provides compositions made from such methods The availability of such materials now makes it possible to produce nanocomposite that exhibit homogeneous nanoscale dispersion of clays and which exhibit excellent improvement in mechanical, thermal, and flame properties. Such nanocomposites can be used in numerous commercial applications.

The invention is further described in the following illustrative examples in which all parts and percentages are by weight unless otherwise indicated.

### EXAMPLES 1-5

Table 2 provides a listing of materials used in Examples 1-5

| **Table 2** | | |
|---|---|---|
| **Designation** | **Description** | **Supplier** |
| Im | Imidazole | Aldrich Chemicals |
| Bim | Benzimidazole | Aldrich Chemicals |
| R-Br | Hexadecyl Biomide, Octadecyl Bromide | Aldrich Chemicals |
| KOH | Potassium Hydroxide | Aldrich Chemicals |
| DMSO | Dimethyl sulfoxide | Aldrich Chemicals |
| THF | Tetrahydrofuran | Aldrich Chemicals |
| Toluene | Toluene | Aldrich Chemicals |
| Methanol | Methanol | Aldrich Chemicals |
| DCM | Dichloromethane | Aldrich Chemicals |
| Delhte HPS clay | Montmorillonite-layer silicate clay with Cationic Exchange Capacity (CEC) = 128.96 mmol/100g | Laviosa Chimica Mineraria |

### Experimental Procedures

This section describes the preparation of imidazolium modified Montmonilonites (Dellite HPS clay). The preparation of the modified clays was performed in two steps. The first step of the preparation of modified clays was the synthesis of the imidazolium salt. The second step was the exchange of the imidazolium ion in the clay.

General procedure for preparing N-Alkylazoles: Potassium hydroxide (0.15 mol), was added to a solution of 0.1 mol of imidazole or benzimidazole in 200 ml of DMSO, the mixture was stirred for 30 min at 70°C, and 0.105 mol of the corresponding alkyl bromide was added drop wise under vigorous stirring. After a night the mixture was cooled to room temperature, 50 ml of distilled water are added, and the alkyl azole precipitates as a pale yellow solid. The salt was filtered out and washed 4 times with 500 ml of distilled water. The product was finally dried.

General procedure for preparing 1,3-Dialkylazolium halides: Alkyl bromide (0.105 mol), was added drop wise under vigorous stirring to a mixture of 0.1 mol of 1-alkylimidazole or 1-alkylbenzimidazole in 200 ml of anhydrous toluene. The mixture was stirred overnight at reflux temperature, and then the solvent was distilled off. The residue was washed 4 times with THF, the suspension was filtered out and dried in a oven under reduced pressure.

Exchange of the imidazolium ion in the clay: The preparation of alkyl-imidazolium montmorillonite consists of a cation-exchange reaction between the montmorillonite-layer silicate (Dellite HPS by Laviosa Chimica Mineraria CEC = 128.96 mmol/100g) and excess of alkyl-imidazolium salt (40% on respect the exchange capacity of the host). The salt was dissolved in methanol at 60°C, and then was added drop wise at an aqueous suspension of montmorillonite (1 wt.%). The mixture was stirred for 5h at 60°C at room temperature overnight. The imidazolium-exchanged montmorillonite was collected by filtration and washed with 1 liter of deionized water (10 x 100 ml) to remove all residual anions. The product was then dried at room temperature and then under vacuum at 100°C overnight, pulverized and purified 5 times with dichloromethane. The characterization of the modified clay was carried out by TGA and XRD analysis.

### Instrumental

The thermogravimetric analysis (TGA) was performed using a Perkin-Elmer TGA7 thermobalance under nitrogen atmosphere (gas flow 40 ml/min) at 10°C/min heating rate from 40°C to 900°C.

The Wide Angle X-ray Scattering (WAXS) data were collected with an X'PertPro diffractometer, equipped with a copper anode (*K*_{α} radiation, λ = 1.5418 Å). The data were collected in the 2θ range 5°-60° by means of an X'Celerator detector.

### EXAMPLE 1

A first purpose of Example 1 was to obtain organic clays with d-spacing of clay intercalates with at least 28Å and thermal stability of 350°C.

A second purpose of Example 1 was to make an N,N-dihexadecyl-Imidazolium (D-2AI) modified clay. The general procedure described above was practiced, except that imidazole and hexadecyl bromide were used as reactants. The result of synthesis was the following specific organic modifier with an imidazolium having two C₁₆ alkyl chains. The chemical structure obtained is shown below in formula III.

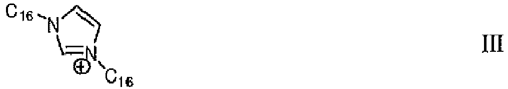

Table 3 summarizes the results of Example 1.

| **Table 3** | | | |
|---|---|---|---|
| Clay | d-spacing (Ǻ) from X-ray analysis | TGA analysis | |
| | | Tₒₙₛₑₜ (°C) | Tₚₑₐₖ (°C) |
| D-2AI (Imidazolium 2C16) | 31.31 | 353 | 428 |

The results of Example 1 indicate that it was possible to make an organic modified clay with imidazolium surfactant to obtain organic clays with d-spacing of clay intercalates with at least 28Å and thermal stability of 350°C from TGA analysis.

### EXAMPLE 2

The purpose of Example 2 was to obtain benzimidazole modified organic clay with d-spacing of clay intercalates with at least 28Å and thermal stability of 350°C.

The procedure described above was practiced with the following specific parameters:

Hexadecyl Bromide and Benzimidazole were used as reactants. The chemical structure of the modifier N,N-dihexadecyl-benzimidazolium (D-2AB, C16) is shown below in formula IV.

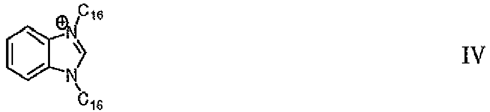

Table 4 summarizes the results of Example 2.

| **Table 4** | | | |
|---|---|---|---|
| Clay | d-spacing (Ǻ) from X-ray analysis | TGA analysis | |
| | | Tₒₙₛₑₜ (°C) | Tₚₑₐₖ (°C) |
| D-2AB (Benzimidazolium 2 C16) | 32.46 | 367 | 445 |

The results of Example 2 indicate that it was possible to make an organic modified clay with imidazolium surfactant to obtain organic clays with d-spacing of clay intercalates with at least 28Å and thermal stability of 350°C from TGA analysis.

### EXAMPLE 3

The procedure described above was practiced with the following specific parameters:

Octadecyl Bromide and Benzimidazole were used as reactants. Table 5 summarizes the results of Example 3.

| **Table 5** | | | |
|---|---|---|---|
| Clay | d-spacing (Ǻ) from X-ray analysis | TGA analysis | |
| | | Tₒₙₛₑₜ (°C) | Tₚₑₐₖ (°C) |
| D-2AB (Benzimidazolium 2 C18) | 36.01 | at least 350°C** | at least 410°C** |
| ** expected | | | |

The results of Example 3 indicate that it was possible to make an organic modified clay with imidazolium surfactant to obtain organic clays with d-spacing of clay intercalates with at least 28Å. The thermal stability, as measured by the Tₒₙₛₑₜ °C and Tₚₑₐₖ °C in Example 3, was not measured in this experiment because the thermal stability would not he expected to change from a material having a C16 chain (Example 2) to C18 (Example 3).

### EXAMPLE 4 (COMPARATIVE)

The procedure described above was practiced with the specific parameters. Hydrogen tallow organic modifier based on ammonium salt was used to modify this clay.

Table 6 summarizes the results of Example 4.

| **Table 6** | | | |
|---|---|---|---|
| Clay | d-spacing (Ǻ)from X-ray analysis | TGA analysis | |
| | | Tₒₙₛₑₜ (°C) | Tₚₑₐₖ (°C) |
| HT (Ammonium) | 26.78 | 258 | 303 |

The results of Example 4 indicate that it was not possible to make an ammonium-modified clay with d-spacing of clay intercalates of at least 28Å and thermal stability of 350°C from TGA analysis.

### EXAMPLE 5 (COMPARATIVE)

The purpose of comparative Example 5 was to make a N-hexadecyl-Imidazolium (D-AI) modified clay. The general procedure described above was practiced, except that 1-methyl-imidazole and hexadecyl bromide were used as reactants. The result of synthesis was the following specific organic modifier, N-hexadecyl-Imidazolium (D-AI,) with an imidazolium having one C16 alkyl chains as shown below in formula V.

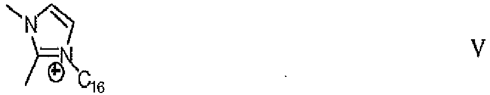

Table 7 summarizes the results of Example 5.

| **Table 7** | | | |
|---|---|---|---|
| Clay | d-spacing (Ǻ) from X-ray analysis | TGA analysis | |
| | | Tₒₙₛₑₜ (°C) | Tₚₑₐₖ (°C) |
| D-/AI (Imidazolium C16) | 21.49 | 350 | 420 |

The results of Example 5 indicate that it was not possible to make an organic modified clay with imidazolium surfactant with one C16 alkyl chain to obtain organic clay with d-spacing of clay intercalates with at least 28Å and thermal stability of 350°C from TGA analysis.

### Summary of Results (Examples 1-5)

Comparative examples E4 (commercial Dellite Clay with ammonium modifier) and E5 (D-AI; with one C16 alkyl chain imidazolium) showed d-spacing lower than desired 28Å. Further, E4 had a thermal stability lower than 300°C (Tₒₙₛₑₜ = 258°C), which was not suitable for making nanocomposites with engineering polymers such as polyesters, polycarbonates, and nylons. However, E5 had Tₒₙₛₑₜ of 350°C, which suits for engineering polymers but as mentioned earlier it has low d-spacing (21.49 Å).

Contrary to the comparative examples E1, E2 and E3 showed excellent thermal stability (Tₒₙₛₑₜ = 350°C) and high d-spacing of > 28 Å. These improved properties are due to the improved clay modification and purification process mentioned in the experimental procedure section.

Table 8 provides a summary of d-spacing and thermal stability of imidazolium and benzimidazolium clays obtained in Examples 1-5.

| **Table 8** | | | | | |
|---|---|---|---|---|---|
| Ex | Clay | | d-spacing (Ǻ) from X-ray analysis | TGA analysis | |
| | | | | Tₒₙₛₑₜ (°C) | Tₚₑₐₖ (°C) |
| 1 | D-2AI (Imidazolium 2C16) | | 31.31 | 353 | 428 |
| 2 | D-2AD (Benzimidazolium 2 C16) | | 32.46 | 367 | 445 |
| 3 | D-2AB (Benzimidazolium 2 C18) | | 3601 | at least 350** | at least 410** |
| 4* | HT (Ammonium) | | 26.78 | 258 | 303 |
| 5* | D-AI (Imidazolium C16) | | 21.49 | 350 | 420 |
| * comparative | | ** expected | | | |

The results shown by Examples 1-3 (E1-E3) indicated that it was possible to make an organic modified clay with imidazolium surfactants to obtain organic clays with d-spacing of clay intercalates with at least 28Å and thermal stability of 350°C from TGA analysis. However, by contrast, the results of Comparative Examples 4 and 5 (E4 and E5) indicated that it was not possible to make an organic modified clay with d-spacing of clay intercalates with at least 28Å and thermal stability of 350°C from TGA analysis.

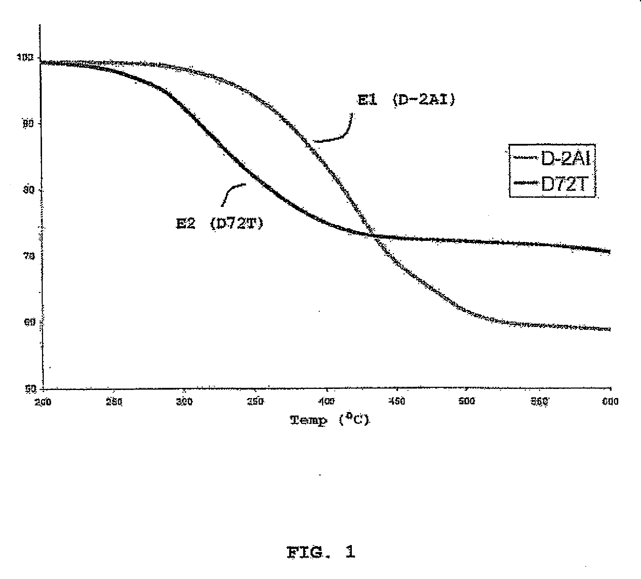

Figures 1-3 further illustrate various embodiments and features of the present invention, and further explain the results summarized in Table 8

Figure 1 shows the thermal stability comparative performance of clays produced in accordance with Example 1 (E1) and in accordance with comparative Example 4 (E4) More specifically, Figure 1 compares the thermal stability of clays modified with N,N-dihexadecyl-Imidazolium (E1, D-2AI) and clays modified with ammonium (E4) surfactants in N₂ at 10°C/min. TGA traces of the imidazolium modified MMT (E1) after purification and commercial alkylammonium modified MMT clay (E4) are shown in Figure 1.

Figure 1 shows that imidazolium clay was stable up to 350°C and was 100°C more stable than the alkylammonium modified MMT. Thus, the imidazolium modified MMT prepared and purified according to the present invention do not show any thermal degradation at the high processing temperatures of polyesters. This data strongly suggests that imidazolium modified clays are suitable for preparation of engineering resins such as polyesters involving high processing temperatures.

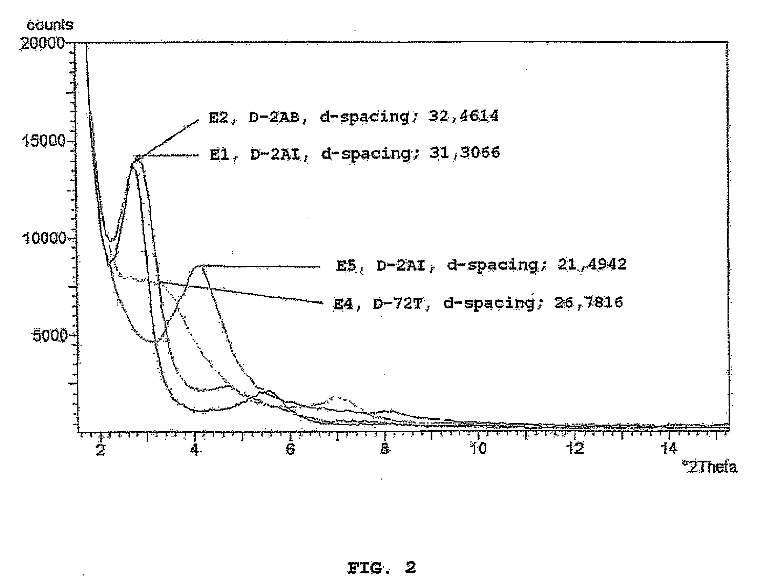

Figure 2 shows the XRD traces of E1, E2, E4, and E5 clays. More specifically, Figure 2 shows X-ray traces of clays modified with 2C16 alkyl imidazolium (E1), clays modified with 2C16 alkyl benzimidazolium (E2), clays modified with commercial ammonium modified clay E4 (Comp), and clays modified with a single C16 alkyl chain imidazolium clay E5 (Comp). The data demonstrates that the presence of the second alkyl chain is necessary in order to obtain d-spacing larger than that of the clay modified with two hydrogenated tallow ammonium salts, i.e., Ditallowdimethylammonium ion with montmorillonite, also known as Dellite^{®} 72T, which is available from Laviosa, Italy. It is possible to observe that the presence of the second alkyl chain on imidazolium or benzimidazolium resulted in d-spacing larger than that of the clay modified with two hydrogenated tallow ammonium modifier (E4). A consistent increase in d-spacing (10Å) was observed by adding the second alkyl chain. No significant differences in d-spacing have been observed between imidazolium and benzimidazolium salts with the same chain lengths.

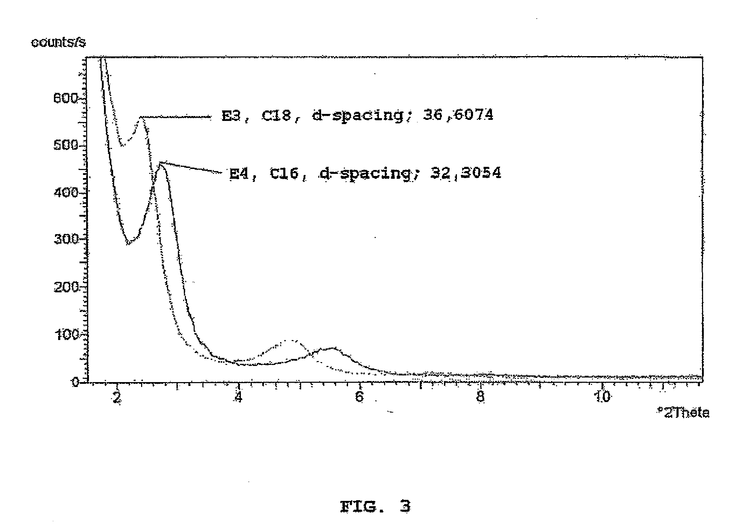

Figure 3 shows a comparison of XRD of D-2AB with two C₁₆ chains and with two C₁₈ chains. More specifically, Figure 3 shows X-ray traces of clays modified with 2C16 alkyl benzimidazolium (E2) and clays modified with 2C18 alkyl benzimidazolium (E3). The use of longer alky chains (C₁₈ as compared to C₁₆) further improves the d-spacing of the clay exceeding 36Ǻ, which is to the best of our knowledge the largest d-spacing reported for alkylimidazolium modified clays.

### EXAMPLES 6-13

The purpose of Examples 6-13 was to make thermoplastic polyester and thermoplastic ionomeric polyester nanocomposites using above claimed high thermal stability and high d-spacing imidazolium and benzimidazolium clays, and further to prove the high molecular weight retention of polyester nanocomposites with claimed thermally stable clays.

Table 9 provides a listing of materials used in nanocomposite preparation.

| **Table 9** | | |
|---|---|---|
| Designation | Description | Supplier |
| Dellite 72T | Commercial ammonium modified Montmorillonite-layer clay | Laviosa Chimica Mineraria |
| Dellite D2AI Clay | N, N-dihexadecyl-Imidazolium modified clay | Synthesized in lab (see expt. Procedure for details) |
| Dellite D-2AB clay | N,N-dihexadecyl-Benzimidazolium modified clay | Synthesized in lab (see expt. Procedure for details) |
| PBT 195 | Poly(1,4-butylene terephthalate) with intrinsic viscosity of 0 7 dl/g as measured in a 60:40 phenol/tetrachloroethane mixture | SABIC Innovative Plastics |
| PBT 3% Telechelic Ionomer | Poly(1,4-butylene terephthalate) telechelic ionomer with 3% ionic groups | SABIC Innovative Plastics |

### Experimental Procedures:

### PBT Nanocomposites with Nanoclays:

Blends of standard and telechelic ionomeric PBT with an ionic group content of 3 mol% (respect to polymer repeating unit) with synthesized nanoclays were prepared by melt mixing in a BRABENDER^{®} Plasticorder 2000 equipped with an electrically-heated mixer. In order to ensure the maximum shearing allowed for by the BRABENDER^{®} mixer, a 10% overfilling of the mixer was kept for all the mixing experiments. In addition, the fraction of the polymer melt that solidified around the pressure ram used to close the mixer ensured that moisture did not come into contact with the melt during the mixing process.

The mixer was preheated at 250°C, then 62 g of a clay-polyester dry blend (5.95 wt/wt) was introduced and mixed at 60 rpm. In some experiments, samples were taken after 10 and 20 min in order to evaluate by GPC the Mw decrease during the nanocomposite preparation. After 30 min, the polymer melt was taken out from the mixer with the aid of a spatula and allowed to cool to room temperature in air.

Techniques:

Molecular weights (expressed in equivalent polystyrene) were determined by gel permeation chromatography (GPC), using a Hewlett Packard Series 1100 liquid chromatography instrument equipped with a PL gel 5µ Mixed-C column. A solution of Chloloform with 5% v/v of hexafluoroisopropanol (HFIP) was used as eluent and a calibration plot was constructed with polystyrene standards.

### EXAMPLE 6

The purpose of Example 6 was to make a nanocomposite of N, N-dihexadecyl imidazolium (D-2AI) modified clay and PBT 3% telechelic inomer. The general procedure described above was practiced. The result of melt blending was the nanocomposite 3% ionomeric PBT with C16 dialkyl imidazolium modified nanoclay.

### EXAMPLE 7

The purpose of Example 7 was to make a nanocomposite of N, N-dihexadecyl benzimidazolium (D-2AB) modified clay and PBT 3% telechelic inomer. The general procedure described above was practiced. The result of melt blending was a nanocomposite 3% ionomeric PBT with C16 dialkyl benzimidazolium modified nanoclay.

### EXAMPLE 8

The purpose of Example 8 was to make a nanocomposite of N, N-dihexadecyl benzimidazolium modified clay and PBT 3% telechelic ionomer. The general procedure described above was practiced except that a dry blend of nanoclay and PBT 3% telechelic ionomer (10:90 wt/wt) ratio was used. The result of the melt blending was the nanocomposite of 3% ionomeric PBT with C16 dialkyl benzimidazolium modified nanoclay.

### EXAMPLE 9

The purpose of Example 9 was to make a nanocomposite of N, N-dihexadecyl imidazolium modified clay and standard PBT. The general procedure described above was practiced except that standard PBT was used. The result of the melt blending was the nanocomposite of standard PBT with C16 dialkyl benzimidazolium modified nanoclay.

### Example 10 (Comparative)

The purpose of comparative Example 10 was to make a control example of standard PBT for nanocomposite examples via melt blending process described above. The general procedure described above was practiced except that only standard PBT was used without any clay sample. The result of the melt blending was the control sample of standard PBT for property evaluation.

### EXAMPLE 11 (Comparative)

The purpose of comparative Example 11 was to make a control example of PBT 3% telechelic ionomer for nanocomposite examples via melt blending process described above. The general procedure described above was practiced except that only PBT 3% telechelic ionomer was used without any clay sample. The result of the melt blending was the control sample of PBT 3% ionomer for property evaluation.

### EXAMPLE 12 (Comparative)

The purpose of comparative Example 12 was to make a nanocomposite of ammonium modified commercial Dellite 72T clay and standard PBT. The general procedure described above was practiced except that standard PBT and D72T was used. The result of the melt blending was the nanocomposite of standard PBT with D72T nanoclay.

### EXAMPLE 13 (Comparative)

The purpose of comparative Example 13 was to make a nanocomposite of ammonium modified commercial Dellite 72T nanoclay and PBT 3% telechelic ionomer. The general procedure described above was practiced except that a dry blend of D72T nanoclay and PBT 3% telechelic ionomer (5:95 wt/wt) ratio was used. The result of the melt blending was the nanocomposite of 3% ionomeric PBT with ammonium modified D72T nanoclay.

### Summary of Results of Examples 6-13

The molecular weight results of nanocomposites based on standard PBT and 3% telechelic PBT using different types of clays are shown in Table 10. Two comparative examples (E10 and E12) were used for regular PBT nanocomposites. E12 nanocomposite with standard PBT and commercial ammonium clay (D72T with Tₒₙₛₑₜ of 258°C) showed only 86% Mw retention Vs standard PBT control E10. However, E9 (Comp) nanocomposite with standard PBT and C16 dialkyl imidazolium nanoclay (Dellite D-2AI clay; Tₒₙₛₑₜ of 353°C) showed up to 98% Mw retention Vs standard control E10 and E12 after blending for 10 minutes in a BRABENDER^{®}. This excellent retention of Mw can be correlated to high thermal stability of Dellite D-2AI clay.

Similar results were also obtained with 3% telechelic PBT ionomer based nanocomposites. Two comparative examples (E11 and E13) were used for regular PBT 3% ionomeric nanocomposites. E6 and E7 nanocomposites with PBT 3% ionomeric PBT and thermally stable (D-2AI and D-2AB) clays at 5 wt% loadings showed molecular weight retention of > 98% Vs control E13 with ammonium modified commercial D72T clay. Using 10 wt% of clay (E8) gave a slight drop in Mw retention (92%), which is still higher than control E13. The final composites all present a thermal stability of 7-15°C higher compared to the starting polymers.

Table 10 summarizes the results of nanocomposites of PBT with 3% ionic groups prepared in BRABENDER^{®} at 260°C for 10 minutes with various nanoclays.

| **Table 10** | | | | | | | | |
|---|---|---|---|---|---|---|---|---|
| Component | E6 | E7 | E8 | E9 | E10 (comp) | E11 (comp) | E12 (comp) | E13 (comp) |
| PBT 3% Ionomer (wt %) | 95 | 95 | 90 | | | 100 | | 95 |
| Dellite D-2AI Clay (wt %) | 5 | | 10 | 5 | | | | |
| Dellite D-2AB Clay (wt %) | | 5 | | | | | | |
| PBT 195 (wt %) | | | | 95 | 100 | | 95 | |
| Dellite 72T Clay (wt %) | | | | | | | 5 | 5 |
| Molecular Weight (Mw) (gm/mole) | 67000 | 68200 | 62460 | 54300 | 55200 | 67700 | 47400 | 55000 |
| % Retention in Mw (%) | 99 | 101 | 92 | 98 | n/a | n/a | 86 | 81 |

The mechanical properties (by DMTA analysis) and the morphology (by TEM) of the nanocomposites prepared using thermally stable imidazolium modified clays do. not differ from those of the nanocomposite obtained with ammonium modified clays, since also using the imidazolium and benzimidazolium organic modified clays a mainly exfoliated morphology and a consistent increase in heat distortion temperature were obtained.

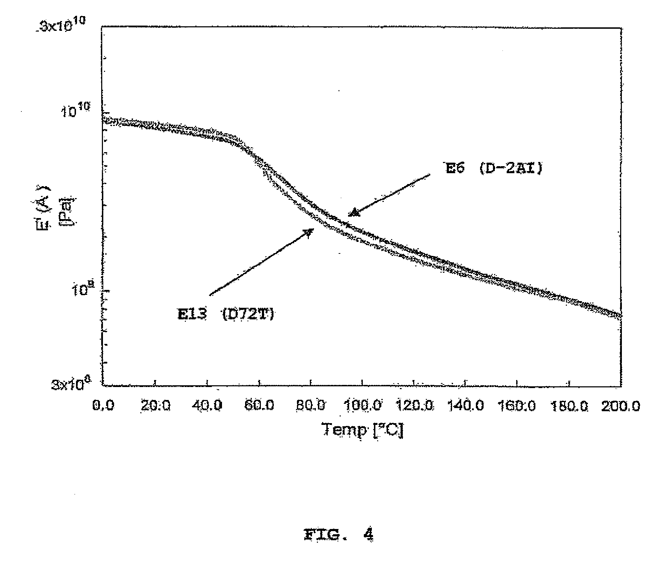

For example, Figure 4 reports the storage modulus measured by DMTA of a nanocomposite of 2% sulfonated telechelic PBT with clays modified with ammonium (Dellite 72T) and imidazolium (D-2AI) surfactants. More specifically, Figure 4 provides a comparison of storage moduli of nanocomposites obtained using clays modified with imidazolium (E6, D-2AI) and ammonium (E13, D72T) clays.

As shown in Figure 4, a consistent improvement in thermo-mechanical properties has been achieved using the imidazolium clays. The use of imidazolium surfactants produces materials, which are less brittle as compared to the nanocomposites, obtained using conventional ammonium salts. Moreover, the use of imidazolium surfactants produces materials, which have a higher retained molecular weight even when subjected to physical stress such as in a BRABENDER® mixer, a multi-screw extruder, and other mixing equipment.

The related art references discussed above are hereby incorporated by reference, in their entirety.

Although the present invention has been described in detail with reference to certain preferred versions thereof, other variations are possible. Therefore, the spirit and scope of the appended claims should not be limited to the description of the versions contained therein.

## Claims

1. A composition comprising a semicrystalline thermoplastic and a dispersed phase of an organically modified clay, wherein the clay is organically modified with at least one benzimidazolium salt of formula 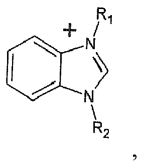 or a imidazolium salt of formula 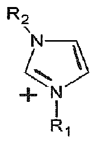 wherein R₁ and R₂, which may be the same or different, are each a C₁₂-C₂₅ alkyl group such that the modified clay has a d-spacing of clay intercalates of at least 28A and thermal stability at 300°C.

2. The composition of claim 1, wherein R₁ and R₂, which may be the same or different, are each a C₁₄-C₂₀ alkyl group.

3. The composition of claim 1 or 2, wherein R₁ and R₂ are a C₁₆ alkyl group.

4. The composition of any of claims 1-3, wherein the d-spacing is greater than 30Å.

5. The composition of any of claims 1-2 or 4, wherein R₁ and R₂ are each C₁₈ alkyl groups and the organically modified clay has a d-spacing of at least 36A.

6. The composition of any of claims 1-5, wherein the semicrystalline thermoplastic is at least one selected from the group consisting of nylon-6, nylon-6,6, poly(butylene terephthalate), poly(ethylene terephthalate), polypropylene, and polyethylene.

7. The composition of any of claims 1-6, wherein the modified clay has a particle size in the range of 1-20 nm.

8. The composition of any of claims 1-7, wherein the semicrystalline thermoplastic is one selected from the group consisting of polyesters and polyester ionomers.

9. The composition of any of claims 1-8, wherein the modified clay is present in an amount of from about 0.5 to about 15% by weight and the thermoplastic is present in an amount of from about 85 to about 99.5% by weight.

10. The composition of claim 8, wherein the polyester ionomers are telechelic.

11. The composition of any of claims 1-10, wherein the clay is one selected from the group consisting of montmorillonite, kaolin, illite, and combinations thereof.

12. A composition comprising a poly(butylene terephthalate) having a dispersed phase of an organically modified clay, wherein the clay is organically modified with an imidazolium salt of the formula 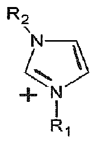 wherein R₁ and R₂, which may be the same or different, are each a C₁₂-C₂₅ alkyl group and wherein the clay is nanosized and has a d-spacing of clay intercalates of at least 30A and a thermal stability of at least 350°C.
